(19) Europäisches Patentamt
European Patent Office
Office européen des brevets

(11) **EP 4 393 911 A1**

(12) **EUROPEAN PATENT APPLICATION**
published in accordance with Art. 153(4) EPC

(43) Date of publication:
**03.07.2024 Bulletin 2024/27**

(21) Application number: **22889382.2**

(22) Date of filing: **03.11.2022**

(51) International Patent Classification (IPC):
**C07D 307/46** (2006.01)

(52) Cooperative Patent Classification (CPC):
**Y02P 20/584**

(86) International application number:
**PCT/CN2022/129612**

(87) International publication number:
**WO 2023/078361 (11.05.2023 Gazette 2023/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **04.11.2021 CN 202111301873**

(71) Applicant: **Zhejiang Sugar Energy Technologyco.,
Ltd.**
**Ningbo, Zhejiang 315201 (CN)**

(72) Inventors:
• **ZHANG, Jian**
  **Ningbo, Zhejiang 315201 (CN)**

• **YANG, Yong**
  **Ningbo, Zhejiang 315201 (CN)**
• **JIN, Haitao**
  **Ningbo, Zhejiang 315201 (CN)**
• **CHEN, Chen**
  **Ningbo, Zhejiang 315201 (CN)**
• **FANG, Qianquan**
  **Ningbo, Zhejiang 315201 (CN)**
• **LU, Guowen**
  **Ningbo, Zhejiang 315201 (CN)**

(74) Representative: **Bayramoglu et al.
Mira Office
Kanuni Sultan Süleyman Boulevard 5387
Street Beytepe, floor 12, no:50
06800 Cankaya, Ankara (TR)**

(54) **METHOD FOR PREPARING 5-HYDROXYMETHYLFURFURAL**

(57) The present invention discloses a method for preparing 5-hydroxymethylfurfural. The method comprises: mixing materials containing a salt solution, a saccharide compound and a catalyst, and subjecting same to a reaction I so as to obtain 5-hydroxymethylfurfural, wherein the pressure of the reaction I is -0.1 to -0.015Mpa. In the preparation method of the present application, the yield of HMF can reach 99%; moreover, the adjustment of the pressure of the reaction system is beneficial to obtaining a relatively high conversion rate of reactants and a relatively high selectivity of a reaction product.

EP 4 393 911 A1

## Description

### TECHNICAL FIELD

The present disclosure relates to a method for preparing 5-hydroxymethylfurfural and belongs to the field of chemical materials.

### BACKGROUND

With the over-exploitation and over-utilization of fossil fuels, the whole world is facing a severe energy crisis and environmental pollution. In order to alleviate these problems, the development of clean and recyclable biomass fuels as an alternative to fossil fuels is a major hotspot in current research. HMF, 5-hydroxymethylfurfural, as a platform compound, plays an important role in the field of biomass energy, and through its reduction and oxidation, a large number of derivatives, such as DMF, BHMF, etc., can be obtained, which can act as an alternative to fossil fuels to alleviate the current energy crisis. HMF is produced by dehydration of biomass and contains a hydroxymethyl group, an aldehyde group and a furan ring in the molecule, which is chemically active and can be used to prepare a variety of derivatives by condensation, hydrogenation and oxidation, etc. HMF is considered to be a versatile chemical intermediate and can be used in the synthesis of plastics, pharmaceuticals, fine chemicals and liquid fuels.

Currently, the main way of HMF preparation is to catalyze the dehydration of saccharide by catalysts at atmospheric pressure, commonly used in aqueous systems, organic solvent systems or aqueous/organic solvent biphasic systems, as well as ionic liquids, with the reaction essentially carried out at higher temperature, which leads to a higher probability of obtaining a higher rate of fructose conversion to HMF, but inevitably leads to an increase in the number of substances (including humins) that are not wanted as well as to the complexity and expense of the method. WO2015113060A2 discloses a method for reacting fructose, water, an acid catalyst and at least one other solvent at 80-180°C in 60 min to give an HMF yield of no more than 80%. Upon completion of the specified conversion rate, the reaction components should be cooled immediately to minimize the formation of unwanted by-products. The method disclosed in this literature with the step of obtaining a relatively low specified HMF yield at the partial conversion endpoint and thereafter quenching the conversion of fructose to HMF at the partial conversion endpoint increases the complexity of the operation. In addition to the separation of HMF, it also includes the separation of HMF from the product mixture containing raw materials or by-products of HMF synthesis and hinders the preparation of pure HMF, resulting in complex and difficult separation of products as well as low selectivity. In order to avoid the disadvantages caused by high temperature, molecular solvents have been tried for biomass conversion reactions. However, the solvents used in these systems are not green, the yield of 5-hydroxymethylfurfural is not high and the reaction takes a long time. Therefore, it is urgent to develop a green and efficient recyclable system with the advantages of good thermal stability, high solubility, low vapor pressure, and low environmental pollution, as well as a temperature controllable process route.

### SUMMARY

It is an object of the present invention to provide a simple method for preparing HMF under pressure from saccharide with basic structural units of hexose, in which the process is mild in conditions, environmentally friendly, and the catalyst can be reused, in order to overcome the drawbacks of the prior art such as high cost, high energy consumption, severe coking, many by-products, poor HMF selectivity, and low yield.

According to an aspect of the present application, a method for preparing 5-hydroxymethylfurfural is provided by mixing a material containing a salt solution, a saccharide compound and a catalyst, and subjecting same to a reaction I so as to obtain 5-hydroxymethylfurfural, wherein a pressure of the reaction I is -0.1- -0.015Mpa, and a reaction time is 0.5~8h.

Preferably, the pressure of the reaction I is -0.08- -0.04Mpa, and the reaction time is 1.5~5h.

Optionally, the reaction I comprises a reaction II-1 and a reaction II-2;

a pressure of the reaction II-1 is -0.045~ -0.015Mpa, and a reaction time of the reaction II-1 is 0~1.5h;
a pressure of the reaction II-2 is -0.1~ -0.045Mpa, and a reaction time of the reaction II-2 is 0.5~6.5h.

Preferably, the pressure of the reaction II-1 is -0.04~ -0.025 Mpa, and a reaction time of the reaction II-1 is 0.5~1.5h;
the pressure of the reaction II-2 is -0.08- -0.065Mpa, and a reaction time of the reaction II-2 is 1~3.5h.

As a green solvent as well as a phase transfer catalyst, the salt solution has been widely studied and applied due to its own characteristics such as high boiling point, easy to extract and easy to recycle. Especially, the application of quaternary ammonium salt liquid solvents to the reaction of HMF preparation from biomass is a hot spot of research in recent years. High reaction temperature at atmospheric pressure leads to serious coking. Therefore, the present invention controls the reaction of materials in the system through the negative pressure conditions in the reaction process to prevent coking by-products produced by excessive temperature; optimize the process to reduce pressure in stages to improve the efficiency of the reaction as well as the selectivity of the target product; and this step is of great significance for the simplification of the process route in the actual production process, the reduction of the cost, as well as the sustainable cycle of production.

Optionally, the salt of the salt solution comprises one or more of an inorganic salt, and an organic salt.

Preferably, the salt solution of reaction I is an inorganic salt solution.

Optionally, the inorganic salt is a metal salt.

Optionally, the metal comprises at least one of sodium, potassium, lithium, magnesium, zinc, calcium.

Optionally, the metal salt comprises at least one of a chloride, a carbonate, a bicarbonate, a sulfate, a bisulfate of the metal.

Optionally, the metal salt is a chloride of the metal.

Optionally, the chloride of the metal comprises at least one of sodium chloride, potassium chloride, lithium chloride, calcium chloride, magnesium chloride, zinc chloride.

Optionally, the metal salt comprises at least one of sodium bromide, sodium carbonate, sodium bicarbonate, sodium sulfate, sodium bisulfate, potassium bromide, potassium chloride, potassium carbonate, lithium bromide, magnesium sulfate.

Preferably, the salt solution of reaction II is selected from inorganic salt solutions.

Optionally, the organic salt comprises at least one of imidazole ionic liquids, pyridine ionic liquids, and quaternary ammonium salt ionic liquids.

Optionally, the organic salt comprises at least one of an alkyl ammonium salt halide, a short chain alkyl imidazole ionic liquid and an alkyl pyridine ionic liquid.

Optionally, the salt solution comprises a solvent; the solvent comprises at least one of water, an organic solvent.

Optionally, the organic solvent comprises at least one of an alcohol compound, a ketone compound, a sulfone compound, an ester compound, an alkyl halide compound, and an amide compound.

Optionally, the organic solvent comprises at least one of 2,5-tetrahydrofuran dimethyl alcohol, methyl isobutyl ketone, dimethyl sulfoxide, n-butanol, 2-butanol, ethyl acetate, dichloromethane, N,N-dimethylacetamide, pyrrolidone, chloroform, acetone.

Optionally, the volume ratio of water to the organic solvent is 5~10:1.

Optionally, the salt in the salt solution: the solvent= 1~10:1.

Optionally, the saccharide compound comprises at least one of monosaccharides, disaccharides, oligosaccharides and polysaccharides.

Optionally, the monosaccharides, the disaccharides, the oligosaccharides and the polysaccharides independently contain basic structural units of hexose.

Optionally, by mass, the salt solution: the saccharide compound: the catalyst =5-40:1-30:0.1.

Optionally, the catalyst comprises at least one of an acid catalyst.

Optionally, the acid catalyst is selected from a group consisting of solid acid catalysts.

Optionally, the solid acid catalyst is selected from acidic ion exchange resins.

Optionally, the acidic ion exchange resin is selected from a group consisting of Nafion-H, Nafion NR50, Dowex 50W-X8, Amberlite-IR 120, Amberlyst 15, Amberlyst 70 acidic ion exchange resin.

Optionally, the catalyst is reusable after roasting.

In the present method, the process method with one-step pressure conditions is simple to operate, and the HMF yield has reached 60-99%. However, the reaction process requires large energy consumption, the pressure conditions are harsh, and the reaction progress is not well controlled. Preferably, the pressure reaction conditions can be reduced to -0.08- -0.04Mpa, and the reaction yield is stable at 90~99% with less by-products in this pressure range after continuous attempts. Optionally, the pressure conditions are carried out in gradients, and the progress of the reaction is controlled by controlling the pressure. At low pressure, a small amount of water is fractionated; increasing the pressure, an acidic aqueous solution containing by-products with low boiling point is fractionated; and at high pressure, a sharp rise of liquid temperature in the reaction is suppressed to prevent the production of humin, a blackening substance. During the reaction process, the salt solution was used to increase the hydrogen ion concentration or as a phase transfer catalyst to accelerate the separation of the target product and the reaction system in two mutually immiscible solvents (liquid-liquid two-phase system or solid-liquid two-phase system), avoiding the consumption of HMF by hydration in the aqueous phase and inhibiting the generation of side reactions, so as to realize the efficient catalytic dehydration of fructose and efficient conversion of 5-hydroxymethylfurfural.

The specific preparation methods of the present application are as follows:

Scheme 1: Heat and stir the salt solution in the reactor, add the saccharide and catalyst. The ratio of the quantity of materials in the system, the salt solution: saccharide: the catalyst = 5~40:1~30:0.1, the system materials are fully contacted by stirring during the reaction, and adjust the pressure of the reaction system to be -0.1~-0.015MPa, the reaction temperature to be 50~100°C, and the reaction time to be 0.5~8h.

Scheme 2: Heat and stir the salt solution in the reactor, add the saccharide and catalyst. The ratio of the quantity of materials in the system, the salt solution: saccharide: the catalyst = 5~40:1-30:0.1, the system materials are fully contacted by stirring during the reaction, and the pressure of the reaction system is adjusted to -0.045~ -0.015MPa, to conserve the starting saccharide or at least one of the two or more starting saccharide to form HMF and thereby form a mixture of products, where the reaction temperature is 50~100°C and the reaction time is 0.5~1.5h.

The pressure of the second stage reaction system is adjusted to -0.1--0.045 MPa, where the reaction temperature is 50~150°C, and the reaction time is 0.5~6.5h. The conversion rate of saccharide and HMF reaction yield were detected.

After the catalyst was separated and washed, it was roasted at 350~850°C for 3-12 h. After roasting, the catalytic performance of the roasted catalyst was evaluated according to the mass ratio of liquid solvent: saccharide: catalyst = 5-40:1-30:0.1.

**Relative to the prior art, the present invention has the following beneficial effects:**

1 Compared with the traditional method of preparing HMF by atmospheric pressure dehydration, the present invention controls the progress of the reaction by controlling the pressure, fractionates water as well as low-boiling point by-products in time, and match the type of salt solution distinctively for one-step decompression and two-step decompression, improves the distribution ratio of the product in the reaction phase and the aqueous phase, avoids the degradation of HMF in the aqueous phase by hydration with water, and also reduces the production of by-products, such as blackening substance, thereby reduces the deactivation of catalyst, has good selectivity for HMF, reduces by-products, is easy to separate, and improves the conversion rate of reactants and HMF yield. It is friendly to the environment and not easy to cause equipment corrosion.

2 In the reaction, salt and natural solvent water, organic solvents or mixtures of water and organic solvents are used as the reaction medium to increase the acid density of the catalyst, strengthen the ionization degree of the catalyst, and enable the reaction of substances in two solvents (liquid-liquid two-phase or solid-liquid two-phase system) that are immiscible with each other. The process is green and pollution-free, the operating conditions are mild, and the process is simple and low-cost.

3 The present invention can directly prepare key intermediates for biomass energy or biomass materials using biomass feedstock as reactants, with a green process, high yield, and a very similar production process to the current petrochemical process, which has a great industrialization prospect and strategic significance.

In conclusion, in the negative pressure reaction process of the present invention, HMF has high selectivity in addition to the salt solution used is environmentally friendly, the operating conditions are mild, the process is simple, the reuse of catalysts reduces the cost, which provides a new way for the industrial production of HMF from a biomass saccharide source, is conducive to the promotion of biomass as a raw material for the preparation of petroleum-based chemicals and the substitution of fuels, and is of great significance for industrialization.

Therefore, the present invention controls the reaction of materials in the system through the negative pressure conditions in the reaction process to prevent coking by-products produced by excessive temperature; optimize the process to reduce pressure in stages to improve the efficiency of the reaction as well as the selectivity of the target product; and this step is of great significance for the simplification of the process route in the actual production process, the reduction of the cost, as well as the sustainable cycle of production.

Based on this, the present invention provides a novel process method for preparing HMF under reduced pressure. The method is characterized by mild conditions, few by-products, 70-100% saccharide conversion and 75-99% HMF reaction yield. The efficient conversion of 5-hydroxymethylfurfural was catalyzed by the dehydration of fructose due to the fractionation of a small amount of water, an acidic aqueous solution containing low-boiling-point by-products, the passage of an inert carrier gas to bring out the volatile by-products, as well as the increase of the concentration of hydrogen ions by using salt solution during the decompression. Through high negative pressure, the sharp rise of temperature within the reaction is inhibited, which slows down the reaction point and effectively prevents the production of the blackening substance humin. In order to improve the selectivity of the saccharide, the present invention is preferred to use the liquid formed by salt and water, organic solvents, a mixture of water and organic solvents, with good mobility, good dispersion, which improve catalyst acid density, and strengthen catalyst ionization, and makes the conversion rate of up to 100% in the reaction of dehydration of saccharide. It also has the advantages of high boiling point, easy to recycle, easy to extract and recyclable. As well as being a liquid solvent, it is pollution-free to the environment, which is conducive to the subsequent separation of products, thus simplifying the process route and improving the yield of the target product.

## DETAILED DESCRIPTION OF THE EMBODIMENTS

The present disclosure will be further described below through specific examples, and the examples are merely exemplary rather than restrictive.

Unless otherwise specified, the saccharide ingredients and chemicals in embodiments of this application are all commercially purchased.

| saccharide ingredients | purity | the source (of a product) |
|---|---|---|
| crystalline fructose | ≥99.5% | Shandong Xiwang Sugar Industry Co., Ltd |
| glucose | ≥99.5% | Shandong Xiwang Sugar Industry Co., Ltd |
| sucrose | ≥99.5% | Shanghai Aladdin Biochemical Technology Co., Ltd. |
| fructose syrup | ≥99.5% | Shandong Xiwang Sugar Industry Co., Ltd |
| inulin | ≥99.5% | Shanghai Aladdin Biochemical Technology Co., Ltd. |
| cellulose | ≥99.5% | Shanghai Aladdin Biochemical Technology Co., Ltd. |

In the embodiments, X-ray powder diffraction of the catalyst samples was performed using a D8 ADVANCE type powder diffractometer.

The products in the synthesis reaction of 5-hydroxymethylfurfural were analyzed by a high performance liquid chromatograph (HPLC) model 1260 from Agilent and quantified by external standard method.

The conversion of 5-hydroxymethylfurfural was calculated based on carbon moles:

$$\text{Yield} = \text{Actual output} / \text{Theoretical output} \times 100\%$$

$$\text{Conversion rate} = \text{amount of reaction/total amount} \times 100\%$$

$$\text{Selectivity} = \text{yield/conversion rate} \times 100\%$$

**Example 1:**

In the reactor, the configured salt solution of potassium chloride: water=3: 1 was put into the reactor, heated to 80°C, stirred well, and crystalline fructose: salt solution: Nafion-H solid acid catalyst=10:3:0.1 was added. Heating and stirring to make the material of the system in full contact during the reaction process.

Adjust the pressure of the reaction system, in which the reaction temperature was 80°C and the reaction time was 5h.

| Pressure | conversion rate of saccharide | HMF reaction yield |
|---|---|---|
| -0.015 | 100% | 78.3% |
| -0.025 | 100% | 78.5% |
| -0.035 | 100% | 79.8% |
| -0.045 | 100% | 85.5% |
| -0.04 | 100% | 92.3% |
| -0.065 | 100% | 97.5% |
| -0.075 | 100% | 95.7% |

(continued)

| Pressure | conversion rate of saccharide | HMF reaction yield |
|---|---|---|
| -0.08 | 100% | 90.8% |
| -0.085 | 100% | 87.3% |
| -0.095 | 100% | 83.1% |
| -0.1 | 100% | 80.1% |

**Example 2:**

In the reactor, the configured salt solution of potassium chloride: water=3: 1 was put into the reactor, heated to 80°C, stirred well, and crystalline fructose: salt solution: Nafion-H solid acid catalyst=10:3:0.1 was added. Heating and stirring to make the material of the system in full contact during the reaction process.

Adjust the pressure of the first stage of the reaction system, in which the reaction temperature was 80°C and the reaction time was 1h.

Adjust the pressure of the second stage of the reaction system, in which the reaction temperature was 80°C and the reaction time was 4h. The conversion rate of saccharide and HMF reaction yield were detected.

| pressure of the first stage | pressure of the second stage | conversion rate of saccharide | HMF reaction yield |
|---|---|---|---|
| -0.015 | -0.045 | 100% | 79.1% |
| -0.025 | -0.055 | 100% | 79.5% |
| -0.035 | -0.065 | 100% | 94.6% |
| -0.045 | -0.075 | 100% | 84.1% |
| -0.045 | -0.08 | 100% | 84.9% |
| -0.045 | -0.095 | 100% | 82.5% |
| -0.025 | -0.065 | 100% | 90.7% |
| -0.035 | -0.075 | 100% | 98.3% |
| -0.025 | -0.08 | 100% | 94.1% |
| -0.04 | -0.075 | 100% | 96.7% |
| -0.04 | -0.08 | 100% | 97.0% |

**Example 3:**

In the reactor, the configured salt solution of salt: water=3: 1 was put into the reactor, heated to 80°C, stirred well, and crystalline fructose: salt solution: Nafion-H solid acid catalyst=10:3:0.1 was added. Heating and stirring to make the material of the system in full contact during the reaction process.

Adjust the pressure of the reaction system was -0.035MPa, the reaction temperature was 80°C and the reaction time was 1h.

Adjust the pressure of the second stage of the reaction system was -0.075MPa, the reaction temperature was 80°C and the reaction time was 4h.

| salt | conversion rate of saccharide | HMF reaction yield |
|---|---|---|
| Sodium chloride | 100% | 97.50% |
| Calcium chloride | 100% | 90.50% |
| Sodium sulfate | 100% | 91.30% |
| Potassium bromide | 100% | 94.10% |
| Magnesium sulfate | 100% | 94.30% |

(continued)

| salt | conversion rate of saccharide | HMF reaction yield |
|---|---|---|
| Lithium carbonate | 100% | 92.20% |
| Tetraethyl ammonium bromide | 100% | 95.60% |
| Tetrabutylammonium chloride | 100% | 95.90% |
| 1-Ethyl-3-methylimidazole bromide | 100% | 94.70% |
| 2-Pyridine acetate | 100% | 96.10% |
| 4-Pyridine acetate | 100% | 95.00% |

**Example 4:**

In the reactor, the configured salt solution of sodium chloride: water: solvent=6:1:1 was put into the reactor, heated to 80°C, stirred well, and crystalline fructose: salt solution: Nafion-H solid acid catalyst=10:3:0.1 was added. Heating and stirring to make the material of the system in full contact during the reaction process.

Adjust the pressure of the reaction system was -0.035MPa, the reaction temperature was 80°C and the reaction time was 1h.

Adjust the pressure of the second stage of the reaction system was -0.075MPa, the reaction temperature was 80°C and the reaction time was 4h.

| solvent | conversion rate of saccharide | HMF reaction yield |
|---|---|---|
| 2,5-tetrahydrofuran dimethyl alcohol | 100% | 97.80% |
| methyl isobutyl ketone | 100% | 94.70% |
| dimethyl sulfoxide | 100% | 93.30% |
| n-butanol | 100% | 92.40% |
| 2-butanol | 100% | 92.10% |
| ethyl acetate | 100% | 93.70% |
| dichloromethane | 100% | 94.50% |
| N,N-dimethylacetamide | 100% | 91.60% |
| pyrrolidone | 100% | 94.10% |
| chloroform | 100% | 93.70% |
| acetone | 100% | 94.30% |

**Example 5**

In the reactor, the configured salt solution of sodium chloride: water=3: 1 was put into the reactor, heated to 80°C, stirred well, and crystalline fructose: salt solution: catalyst=10:3:0.1 was added. Heating and stirring to make the material of the system in full contact during the reaction process.

Adjust the pressure of the reaction system was -0.035MPa, the reaction temperature was 80°C and the reaction time was 1h.

Adjust the pressure of the second stage of the reaction system was -0.075MPa, the reaction temperature was 80°C and the reaction time was 4h.

| catalyst | conversion rate of saccharide | HMF reaction yield |
|---|---|---|
| Nafion NR50 | 100% | 97.70% |
| Dowex 50W-X8 | 100% | 96.30% |
| Amberlite-IR 120 | 100% | 96.10% |

(continued)

| catalyst | conversion rate of saccharide | HMF reaction yield |
|---|---|---|
| Amberlyst_x005f- 15 | 100% | 97.50% |
| Amberlyst_x005f-70 | 100% | 98.00% |

The present invention applies the pressure control in the reaction process to the method for catalyzing the preparation of HMF from saccharide, which solves the problem of high temperature and serious coking in the atmospheric pressure reaction, controls the occurrence of side reactions in the reaction process, and improves the selectivity and yield of the target product.

The above examples are merely a few examples of the present disclosure, and do not limit the present disclosure in any form. Although the present disclosure is disclosed as above with preferred examples, the present disclosure is not limited thereto. Some changes or modifications made by any technical personnel familiar with the profession using the technical content disclosed above without departing from the scope of the technical solutions of the present disclosure are equivalent to equivalent implementation cases and fall within the scope of the technical solutions.

**Claims**

1. A method for preparing 5-hydroxymethylfurfural, wherein mix a material containing a salt solution, a saccharide compound and a catalyst, and subject same to a reaction I so as to obtain 5-hydroxymethylfurfural, wherein a pressure of the reaction I is -0.1- -0.015Mpa, and a reaction time is 0.5~8h;

   by mass, the salt solution: the saccharide compound: the catalyst =5-40:1-30:0.1;
   the solid acid catalyst is selected from acidic ion exchange resins.

2. The method according to claim 1, wherein the pressure of the reaction I is -0.08- -0.04Mpa, and the reaction time is 1.5~5h.

3. The method according to claim 1, wherein the reaction I comprises a reaction II-1 and a reaction II-2;

   a pressure of the reaction II-1 is -0.045~ -0.015Mpa, and a reaction time of the reaction II-1 is 0.5~1.5h;
   a pressure of the reaction II-2 is -0.1~ -0.045Mpa, and a reaction time of the reaction II-2 is 0.5~6.5h.

4. The method according to claim 3, wherein the pressure of the reaction II-1 is -0.04~ -0.025 Mpa, and the reaction time of the reaction II-1 is 0.5~1.5h; the pressure of the reaction II-2 is -0.08- - 0.065Mpa, and the reaction time of the reaction II-2 is 1~3.5h.

5. The method according to any one of claims 1 to 4, wherein the salt of the salt solution comprises one or more of an inorganic salt, and an organic salt.

6. The method according to claims 5, wherein the inorganic salt comprises a metal salt.

7. The method according to claims 6, wherein the metal comprises at least one of sodium, potassium, lithium, magnesium, zinc, calcium.

8. The method according to claims 6, wherein the metal salt comprises at least one of a chloride, a carbonate, a bicarbonate, a sulfate, a bisulfate of the metal.

9. The method according to claims 8, wherein the chloride of the metal comprises at least one of sodium chloride, potassium chloride, lithium chloride, calcium chloride, magnesium chloride, zinc chloride.

10. The method according to claims 6, wherein the metal salt comprises at least one of sodium bromide, sodium carbonate, sodium bicarbonate, sodium sulfate, sodium bisulfate, potassium bromide, potassium chloride, potassium carbonate, lithium bromide, magnesium sulfate.

11. The method according to claims 5, wherein the organic salt comprises at least one of imidazole ionic liquids, pyridine ionic liquids, and quaternary ammonium salt ionic liquids.

12. The method according to claims 11, wherein the organic salt comprises at least one of an alkyl ammonium salt halide, a short chain alkyl imidazole ionic liquid and an alkyl pyridine ionic liquid.

13. The method according to any one of claims 1 to 4, wherein by mass, the salt in the salt solution: solvent= 1~10:1.

<table>
<tr><td colspan="2">INTERNATIONAL SEARCH REPORT</td><td colspan="2">International application No.<br>**PCT/CN2022/129612**</td></tr>
</table>

**A. CLASSIFICATION OF SUBJECT MATTER**

C07D 307/46(2006.01)i

According to International Patent Classification (IPC) or to both national classification and IPC

**B. FIELDS SEARCHED**

Minimum documentation searched (classification system followed by classification symbols)

C07D307/-

Documentation searched other than minimum documentation to the extent that such documents are included in the fields searched

Electronic data base consulted during the international search (name of data base and, where practicable, search terms used)

CNTXT, CNKI: 羟甲基, 糠醛, 催化, 离子液体

**C. DOCUMENTS CONSIDERED TO BE RELEVANT**

| Category* | Citation of document, with indication, where appropriate, of the relevant passages | Relevant to claim No. |
|---|---|---|
| Y | CN 101333200 A (HUAIBEI MINSHENG CHEMICAL TECHNOLOGY CO., LTD.) 31 December 2008 (2008-12-31)<br>    embodiment 1, and claims 2-3 | 1-13 |
| Y | CN 101367783 A (UNIVERSITY OF SCIENCE AND TECHNOLOGY OF CHINA) 18 February 2009 (2009-02-18)<br>    description, pages 5-6 | 1-13 |
| PX | CN 113861139 A (ZHEJIANG TANGNENG TECHNOLOGY CO., LTD.) 31 December 2021 (2021-12-31)<br>    claims 1-10 | 1-13 |

☐ Further documents are listed in the continuation of Box C.  ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **10 January 2023** | **18 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/129612**

| Patent document cited in search report | | | Publication date (day/month/year) | Patent family member(s) | | | Publication date (day/month/year) |
|---|---|---|---|---|---|---|---|
| CN | 101333200 | A | 31 December 2008 | CN | 101333200 | B | 09 May 2012 |
| CN | 101367783 | A | 18 February 2009 | US | 2012172607 | A1 | 05 July 2012 |
| | | | | CN | 101619051 | A | 06 January 2010 |
| CN | 113861139 | A | 31 December 2021 | None | | | |

Form PCT/ISA/210 (patent family annex) (January 2015)

## REFERENCES CITED IN THE DESCRIPTION

**Patent documents cited in the description**

- WO 2015113060 A2